(19) Europäisches Patentamt
European Patent Office
Office européen des brevets

(11) **EP 2 114 469 B1**

(12) **EUROPÄISCHE PATENTSCHRIFT**

(45) Veröffentlichungstag und Bekanntmachung des Hinweises auf die Patenterteilung:
**29.03.2017 Patentblatt 2017/13**

(21) Anmeldenummer: **08708785.4**

(22) Anmeldetag: **07.02.2008**

(51) Int Cl.:
**A61L 15/46** (2006.01)    **A61L 15/60** (2006.01)
**C08J 7/14** (2006.01)

(86) Internationale Anmeldenummer:
**PCT/EP2008/051505**

(87) Internationale Veröffentlichungsnummer:
**WO 2008/095983 (14.08.2008 Gazette 2008/33)**

(54) **WASSERABSORBIERENDES POLYMERGEBILDE MIT HOHER AMMONIAK-BINDEKAPAZITÄT**

WATER-ABSORBING POLYMER STRUCTURE HAVING GREAT AMMONIA-BINDING CAPACITY

MODÈLE POLYMÈRE ABSORBANT L'EAU À CAPACITÉ ÉLEVÉE DE LIAISON AVEC L'AMMONIAC

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **09.02.2007 DE 102007007203**

(43) Veröffentlichungstag der Anmeldung:
**11.11.2009 Patentblatt 2009/46**

(73) Patentinhaber: **Evonik Degussa GmbH**
**45128 Essen (DE)**

(72) Erfinder:
• **HARREN, Jörg**
  **47807 Krefeld (DE)**
• **RAMLOW, Stephan**
  **47807 Krefeld (DE)**
• **BUSCH, Axel**
  **47805 Krefeld (DE)**
• **FURNO, Franck**
  **40545 Düsseldorf (DE)**

(56) Entgegenhaltungen:
**EP-A- 0 316 518        EP-A1- 1 140 230
EP-A1- 1 187 640       EP-A1- 1 363 682
WO-A-01/32226          WO-A-01/35885
WO-A-03/002623         WO-A-2007/057203
WO-A-2007/104641       WO-A1-2004/037900
GB-A- 2 093 351        JP-A- H10 298 442
US-A- 3 707 148**

**Beschreibung**

Die vorliegende Erfindung betrifft ein Verfahren zur Herstellung eines wasserabsorbierenden Polymergebildes, die durch dieses Verfahren erhältlichen, wasserabsorbierenden Polymergebilde, wasserabsorbierende Polymergebilde, einen Verbund, ein Verfahren zur Herstellung eines Verbundes, den durch dieses Verfahren erhältlichen Verbund, Schäume, Formkörper, Fasern, Folien, Filme, Kabel, Dichtungsmaterialien, flüssigkeitsaufnehmende Hygieneartikel, Träger für pflanzen- und pilzwachstumregulierende Mittel, Verpackungsmaterialien, Bodenzusätze oder Baustoffe sowie die Verwendung eines wasserabsorbierenden Polymergebildes.

Superabsorber sind wasserunlösliche, vernetzte Polymere, die in der Lage sind, unter Quellung und Ausbildung von Hydrogelen große Mengen an Wasser, wässrigen Flüssigkeiten, insbesondere Körperflüssigkeiten, vorzugsweise Urin oder Blut, aufzunehmen und unter Druck zurückzuhalten. Superabsorber absorbieren vorzugsweise mindestens das 100-fache ihres Eigengewichts an Wasser. Weitere Einzelheiten zu Superabsorbern sind in "Modern Superabsorbent Polymer Technology", F. L. Buchholz, A. T. Graham, Wiley-VCH, 1998" offenbart. Durch diese charakteristischen Eigenschaften sind diese wasserabsorbierenden Polymere hauptsächlich in Sanitärartikeln wie beispielsweise Babywindeln, Inkontinenzprodukten oder Damenbinden eingearbeitet.

Die Herstellung der Superabsorber erfolgt in der Regel durch die radikalische Polymerisation säuregruppen-tragender Monomere in Gegenwart von Vernetzern, wobei diese säuregruppen-tragenden Monomere vor oder auch nach der Polymerisation zumindest teilweise neutralisiert werden.

Bei Hygieneartikeln besteht insbesondere im Bereich der Erwachsenenhygiene ein Bedarf an Superabsorbern mit der Fähigkeit, unangenehme Gerüche zu binden. Derartige geruchsbindende Eigenschaften werden beispielsweise durch den Zusatz geruchsbindender Additive, wie etwa Cyclodextrinen, ermöglicht, wie dies beispielsweise in der WO 01/13841 A1 beschrieben ist. Der Nachteil des Zusatzes geruchsbindender Additive besteht jedoch darin, dass diese in der Regel pulverförmigen Zusätze oftmals zu einem Stauben der wasserabsorbierenden Polymergebilde, insbesondere bei einer Förderung in Anlagen zur Herstellung von Hygieneartikeln, führen. Weitere Möglichkeiten zur Geruchskontrolle in Hygieneartikeln sind beispielsweise in der WO 03/002623 A1 beschrieben.

Wichtig für den Tragekomfort von Hygieneartikeln ist insbesondere die Bindung des Ammoniaks, welcher durch den bakteriellen Abbau von Harnstoff entsteht. Durch ihre chemische Zusammensetzung aus vernetzer, teilneutralisierter Acrylsäure sind Superabsorber in der Lage, Ammoniak durch Säure-Base-Reaktionen zu binden. Durch die Absenkung des Neutralisationsgrades der polymerisierten Acrylsäure und der damit verbundenen Zunahme von protonierten Carbonsäuregruppen lässt sich die Ammoniak-Bindekapazität erhöhen. Der Absenkung des pH-Wertes sind jedoch Grenzen gesetzt, da sich mit abnehmendem Neutralisationsgrad die Gesamtperformance eines superabsorbierenden Polymergebildes verschlechtert. Moderne Superabsorber weisen ein Optimum an Flüssigkeitsaufnahmeperformance bei Neutralisationsgraden im Bereich von 65 bis 80 Mol-% auf.

Bis zu einem Neutralisationsgrad von 50 Mol-% lässt sich noch eine akzeptable Gesamtperformance erzielen. Ein weiterer Nachteil eines Neutralisationsgrades von 50 Mol-% oder weniger besteht darin, dass die Quelleigenschaften durch die geringe Ionizität des Polymernetzwerkes stark herabgesetzt sind.

Um in Hygieneartikeln durch die Senkung des pH-Wertes die geruchsbindenden Eigenschaften weiter zu verbessern, schlägt US 3,794,034 vor, innerhalb des Fasermaterials des Hygieneartikels eine pulverförmige, saure Substanz, wie etwa Zitronensäure, bereitzustellen. Der Einsatz von beispielsweise auf vernetzten Polyacrylaten basierenden Superabsorbern wird in US 3,794,034 nicht offenbart.

WO 00/35502 A1 schlägt vor, zur Verbesserung der geruchsbindenden Eigenschaften eines einen Superabsorber beinhaltenden Hygieneartikels dem absorbierenden Kern eines solchen Hygieneartikels neben dem Superabsorber auch Milchsäure produzierende Bakterien zuzusetzen, so dass nach dem in Kontakt treten des Hygieneartikels mit Körperflüssigkeiten der pH-Wert in einem Bereich von 3,5 bis 5,5 liegt. Der Nachteil eines solchen Hygieneartikels besteht darin, dass zum einen der Zusatz Milchsäure produzierender Bakterien einen zusätzlichen Verfahrensschritt bei der Herstellung der Hygieneartikel erforderlich macht und dass zum anderen, insbesondere bedingt durch die Temperaturempfindlichkeit der Milchsäure produzierenden Bakterien, die geruchsbindenden Eigenschaften solcher Hygieneartikel durch Umwelteinflüsse, insbesondere durch besonders hohe oder tiefe Temperaturen, nachteilig beeinflusst werden können.

WO 01/32226 A1 schlägt vor, zur Verbesserung der geruchsbindenden Eigenschaften eines einen Superabsorber beinhaltenden Hygieneartikels im absorbierenden Kern des Hygieneartikel, vom Superabsorber getrennt, saure Substanzen wie etwa organische Carbonsäuren vorzusehen. Auch hier besteht der Nachteil darin, dass bei der Herstellung des Hygieneartikels zusätzliche Verfahrensschritte erforderlich sind, in denen die saure Komponente in den absorbierenden Kern eingebracht werden muss. Darüber hinaus sind die geruchsbindenden Eigenschaften derartiger Hygieneartikel noch verbesserungswürdig.

WO 03/002623 A1 beschreibt ein Verfahren zur Herstellung geruchsbindender Superabsorber, bei dem schwach teilneutralisierte wasserabsorbierende Polymergebilde mit einem pH-Wert von weniger als 5,7 oberflächennachvernetzt wird. Der Nachteil der in diesem Stand der Technik beschriebenen Superabsorber besteht jedoch darin, dass das Ammoniak-Bindevermögen nur gering ist.

Der vorliegenden Erfindung lag die Aufgabe zugrunde, die sich aus dem Stand der Technik ergebenden Nachteile hinsichtlich der geruchsbindenden Eigenschaften von Superabsorbern beinhaltenden Hygieneartikeln zu verbessern.

Insbesondere lag der vorliegenden Erfindung die Aufgabe zugrunde, wasserabsorbierende Polymergebilde bereitzustellen, welche im Vergleich zu den aus dem Stand der Technik bekannten wasserabsorbierenden Polymergebilden besser in der Lage sind, das Entweichen unangenehm riechender Verbindungen aus Hygieneartikeln zu unterbinden und dennoch zufrieden stellende Absorptionseigenschaften aufweisen.

Darüber hinaus lag der vorliegenden Erfindung die Aufgabe zugrunde, wasserabsorbierende Polymergebilde bereitzustellen, welche gegenüber herkömmlichen Polymergebilden verbesserte geruchsbindende Eigenschaften aufweisen und sich zudem im Vergleich zu diesen herkömmlichen Superabsorbern besser verarbeiten lassen, insbesondere besser in Förderanlagen zur Herstellung von Hygieneartikeln transportieren lassen.

Auch lag der vorliegenden Erfindung die Aufgabe zugrunde, ein Verfahren anzugeben, mit dem derart vorteilhafte wasserabsorbierende Polymergebilde hergestellt werden können.

Der vorliegenden Erfindung lag auch die Aufgabe zugrunde, Verbunde bereitzustellen, welche im Vergleich zu den aus den Stand der Technik bekannten Verbunden verbesserte geruchsbindende Eigenschaften und Absorptionseigenschaften aufweisen und sich zudem im Vergleich zu herkömmlichen Verbunden mit möglichst wenigen Verfahrensschritten herstellen lassen.

Einen Beitrag zur Lösung der vorstehend genannten Aufgaben leistet ein Verfahren zur Herstellung eines wasserabsorbierenden Polymergebildes gemäß den Ansprüchen, umfassend die Verfahrensschritte:

i) Bereitstellen eines unbehandelten, wasserabsorbierenden Polymergebildes mit einem Neutralisationsgrad von höchstens 70 Mol-%, bevorzugt von höchstens 65 Mol-%, noch mehr bevorzugt von höchstens 60 Mol-%, darüber hinaus bevorzugt von höchstens 55 Mol-%, am meisten bevorzugt mit einem Neutralisationsgrad in einem Bereich von 45 bis 55 Mol-%, wobei vorzugsweise ein Neutralisationsgrad von 20 Mol-%, besonders bevorzugt von 30 Mol-%, noch mehr bevorzugt von 40 Mol-% und am meisten bevorzugt von 45 Mol-% nicht unterschritten wird;

ii) in Kontakt bringen des wasserabsorbierenden Polymergebildes mit einer sauren, organischen Komponente.

Überraschender Weise wurde festgestellt, dass durch das Vermischen eines nur schwach neutralisierten, wasserabsorbierenden Polymergebildes mit einer sauren Komponente wasserabsorbierende Polymergebilde erhalten lassen, welche zugleich vorteilhafte, geruchsbindende Eigenschaften und eine vorteilhafte Gesamtperformance aufweisen.

"Unbehandelt" im Sinne der vorliegenden Erfindung bedeutet, dass die im Verfahrenschritt i) bereitgestellten, wasserabsorbierenden Polymergebilde noch nicht mit der sauren, vorzugsweise organischen Komponente in Kontakt gebracht worden sind. Die Bezeichnung "unbehandelt" schließt hingegen nicht aus, dass die wasserabsorbierenden Polymergebilde mittels anderer Oberflächenmodifizierungsmaßnahmen, wie etwa der Oberflächennachvernetzung, modifiziert sein können.

Als im Verfahrensschritt i) bereitgestellte unbehandelte, wasserabsorbierende Polymergebilde sind Fasern, Schäume oder Teilchen bevorzugt, wobei Fasern und Teilchen bevorzugt und Teilchen besonders bevorzugt sind.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Erfindungsgemäß bevorzugte Polymerteilchen sind so dimensioniert, dass sie eine mittlere Teilchengröße gemäß ERT 420.2-02 im Bereich von 10 bis 3000 $\mu$m, vorzugsweise 20 bis 2000 $\mu$m und besonders bevorzugt 150 bis 850 $\mu$m oder 150 bis 600 $\mu$m aufweisen. Dabei ist es insbesondere bevorzugt, dass der Anteil der Polymerteilchen mit einer Partikelgröße in einem Bereich von 300 bis 600 $\mu$m mindestens 30 Gew.-%, besonders bevorzugt mindestens 40 Gew.-% und am meisten bevorzugt mindestens 50 Gew.-%, bezogen auf das Gesamtgewicht der nachvernetzten, wasserabsorbierenden Polymerteilchen, beträgt.

In einer bevorzugten Ausführungsform der im Verfahrensschritt i) bereitgestellten, wasserabsorbierenden Polymergebilde basieren diese auf

($\alpha$1) 20-99,999 Gew.-%, bevorzugt 55-98,99 Gew.-% und besonders bevorzugt 70-98,79 Gew.-% polymerisierten, ethylenisch ungesättigten, säuregruppen-tragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,

($\alpha$2) 0-80 Gew.-%, vorzugsweise 0-44,99 Gew.-% und besonders bevorzugt 0,1-44,89 Gew.-% polymerisierten, monoethylenisch ungesättigten, mit ($\alpha$1) copolymerisierbaren Monomeren,

($\alpha$3) 0,001-5 Gew.-%, vorzugsweise 0,01-3 Gew.-% und besonders bevorzugt 0,01-2,5 Gew.-% eines oder mehrerer Vernetzer,

($\alpha$4) 0-30 Gew.-%, vorzugsweise 0-5 Gew.-% und besonders bevorzugt 0,1-5 Gew.-% eines wasserlöslichen Polymeren,

($\alpha$5) 0-20 Gew.-%, vorzugsweise 2,5-15 Gew.-% und besonders bevorzugt 5-10 Gew.-% Wasser, sowie

($\alpha$6) 0-20 Gew.-%, vorzugsweise 0-10 Gew.-% und besonders bevorzugt 0,1-8 Gew.-% eines oder mehrerer Hilfsmittel, wobei die Summe der Gewichtsmengen ($\alpha$1) bis ($\alpha$6) 100 Gew.-% beträgt.

In diesem Zusammenhang bedeutet die Forderung, wonach die im Verfahrensschritt i) bereitgestellten, unbehandelten, wasserabsorbierenden Polymergebilde einen Neutralisationsgrad von höchstens 70 Mol-%, bevorzugt von höchstens 65 Mol-%, noch mehr bevorzugt von höchstens 60 Mol-%, darüber hinaus bevorzugt von höchstens 55 Mol-% und am meisten bevorzugt einen Neutralisationsgrad in einem Bereich von 45 bis 55 Mol-% aufweisen sollen, dass höchstens 70 Mol-%, höchstens 65 Mol-%, höchstens 60 Mol-% bzw. höchstens 55 Mol-% der Säuregruppen der Monomere ($\alpha$1) als deprotonierte Carboxylat-Gruppen vorliegen.

Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Bevorzugte ethylenisch ungesättigte, säuregruppenhaltige Monomere ($\alpha$1) sind vorzugsweise diejenigen Verbindungen, die in der WO 2004/037903 A2, als ethylenisch ungesättigte, säuregruppenhaltige Monomere ($\alpha$1) genannt werden. Besonders bevorzugte ethylenisch ungesättigte, säuregruppenhaltige Monomere ($\alpha$1) sind Acrylsäure und Methacrylsäure, wobei Acrylsäure am meisten bevorzugt ist.

Gemäß einer Ausführungsform des erfindungsgemäßen Verfahrens werden unbehandelte, wasserabsorbierende Polymergebilde eingesetzt, bei denen die monoethylenisch ungesättigten, mit ($\alpha$1) copolymerisierbare Monomere ($\alpha$2) Acrylamide, Methacrylamide oder Vinylamide sind.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino-(meth)acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Gemäß einer anderen Ausführungsform des erfindungsgemäßen Verfahrens werden wasserabsorbierende Polymergebilde eingesetzt, bei denen die monoethylenisch ungesättigten, mit ($\alpha$1) copolymerisierbare Monomere ($\alpha$2) wasserlösliche Monomere sind. In diesem Zusammenhang sind insbesondere Alkoxypolyalkylenoxid(meth)acrylate wie Methoxypolyethylenglykol(meth)-acrylate bevorzugt.

Des Weiteren sind als monoethylenisch ungesättigte, mit ($\alpha$1) copolymerisierbaren Monomere ($\alpha$2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)-acrylat bevorzugt.

Die monoethylenisch ungesättigten, mit ($\alpha$1) copolymerisierbaren Monomere ($\alpha$2) umfassen weiterhin Methylpolyethylenglykolallylether, Vinylacetat, Styrol und Isobutylen.

Als Vernetzer ($\alpha$3) werden vorzugsweise diejenigen Verbindungen eingesetzt, die in der WO 2004/037903 A2 als Vernetzer ($\alpha$3) genannt werden. Unter diesen Vernetzern sind wasserlösliche Vernetzer besonders bevorzugt. Am meisten bevorzugt sind dabei N,N'-Methylenbisacrylamid, Polyethylenglykoldi-(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat.

Als wasserlösliche Polymere ($\alpha$4) können in den Polymergebilden wasserlösliche Polymere, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsmittel ($\alpha$6) sind vorzugsweise Stellmittel, Geruchsbinder, oberflächenaktive Mittel oder Antioxidatien sowie diejenigen Additive, die zur Herstellung der Polymergebilde eingesetzt wurden (Initiatoren usw.) in den Polymergebilden enthalten.

In einer besonderen Ausführungsform der im Verfahrensschritt i) bereitgestellten, wasserabsorbierenden Polymergebilde basieren diese zu mindestens 50 Gew.-%, vorzugsweise zu mindestens 70 Gew.-% und darüber hinaus bevorzugt zu mindestens 90 Gew.-% auf carbonsäuregruppen- bzw. carboxylatgruppen-tragenden Monomeren.

Aus den vorgenannten Monomeren, Comonomeren, Vernetzern, wasserlöslichen Polymeren und Hilfsstoffen lassen sich die unbehandelten, wasserabsorbierenden Polymergebilde durch verschiedene Polymerisationsweisen herstellen. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135 A1, US 4,076,663, DE 35 03 458 A1, DE 40 20 780 C1, DE 42 44 548 A1, DE 43 33 056 A1, DE 44 18 818 A1.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO 2004/037903 A2 als mögliche Initiatoren genannt werden.

Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt.

Auch die inverse Suspensions- und Emulsionspolymerisation kann zur Herstellung der Polymergebilde angewendet werden. Gemäß diesen Prozessen wird eine wässrige, teilneutralisierte Lösung der Monomeren ($\alpha$1), und ($\alpha$2), gegebenenfalls beinhaltend wasserlösliche Polymere und Hilfsstoffe, mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren ($\alpha$4) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers und/oder durch Reaktion geeigneter Vernetzer mit funktionellen Gruppen des Polymeren während der Polymerisationsschritte erfolgen. Die Verfahren sind beispielsweise in den Veröffentlichungen US 4,340,706, DE 37 13 601 A1, DE 28 40 010 A1 und WO 96/05234 A1 beschrieben.

Die bei der Lösungspolymerisation oder der inversen Suspensions- und Emulsionspolymerisation nach der Polymerisation erhaltenen Hydrogele werden in einem weiteren Verfahrenschritt getrocknet.

Insbesondere im Falle der Lösungspolymerisation ist es jedoch bevorzugt, dass die Hydrogele vor der Trocknung zunächst zerkleinert werden. Dieses Zerkleinern erfolgt durch dem Fachmann bekannte Zerkleinerungsvorrichtungen, wie etwa einem Fleischwolf.

Die Trocknung des Hydrogels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Hydrogels bis zu einem Wassergehalt von 0,5 bis 25 Gew.-%, vorzugsweise von 1 bis 10 Gew.-% erfolgt, wobei die Trocknungstemperaturen üblicherweise in einem Bereich von 100 bis 200°C liegen.

Die nach dem Trocknen erhaltenen wasserabsorbierenden Polymergebilde können, insbesondere dann, wenn sie durch Lösungspolymerisation erhalten wurden, in einem weiteren Verfahrensschritt noch zermahlt und auf eine die eingangs genannte Wunschkorngröße abgesiebt werden. Das Zermahlen der getrockneten, wasserabsorbierenden Polymergebilde erfolgt vorzugsweise in geeigneten, mechanischen Zerkleinerungsvorrichtungen, wie etwa einer Kugelmühle.

Das im Verfahrensschritt i) bereitgestellte, unbehandelte, wasserabsorbierende Polymergebilde ist oberflächennachvernetzt. Oberflächennachvernetzte, wasserabsorbierende Polymergebilde weisen eine Kern-Schale-Struktur auf, wobei die Polymergebilde im Bereich der Schale einen höheren Vernetzungsgrad aufweisen als im Kernbereich.

Bei der Oberflächennachvernetzung werden die getrockneten Polymergebilde oder aber das noch nicht getrocknete, jedoch vorzugsweise bereits zerkleinerte Hydrogel mit einem vorzugsweise organischen, chemischen Oberflächennachvernetzer in Kontakt gebracht. Dabei wird der Nachvernetzer insbesondere dann, wenn er unter den Nachvernetzungsbedingungen nicht flüssig ist, vorzugsweise in Form eines Fluids $F_1$ umfassend den Nachvernetzer sowie ein Lösungsmittel mit den Polymerteilchen bzw. dem Hydrogel in Kontakt gebracht. Geeignete Lösungsmittel sind, neben Wasser, insbesondere mit Wasser mischbare, organische Lösungsmittel wie etwa Methanol, Ethanol, 1-Propanol, 2-Propanol, 1,2-Propandiol, 1,3-Propandiol, 1-Butanol, 2-Butanol, tert.-Butanol, iso-Butanol oder aber Mischungen aus organischen Lösungsmitteln oder Mischungen aus Wasser mit einem oder mehreren dieser organischen Lösungsmittel, wobei Wasser als Lösungsmittel am meisten bevorzugt ist. Weiterhin ist es bevorzugt, dass der Nachvernetzer in dem Fluid $F_1$ in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht des Fluids $F_1$, enthalten ist.

Das in Kontakt bringen des Polymergebildes bzw. des zerkleinerten Hydrogels mit dem Fluid $F_1$ beinhaltend den Nachvernetzer erfolgt im erfindungsgemäßen Verfahren vorzugsweise durch gutes Vermischen des Fluids $F_1$ mit dem Polymergebilde, wobei geeignete Mischaggregate zum Aufbringen des Fluids $F_1$ wiederum der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer), sind.

Bei der Nachvernetzung wird das wasserabsorbierende Polymergebilde vorzugsweise mit höchstens 20 Gew.-%, besonders bevorzugt mit höchstens 15 Gew.-%, darüber hinaus bevorzugt mit höchstens 10 Gew.-%, darüber hinaus noch mehr bevorzugt mit höchstens 5 Gew.-% an Lösungsmittel, vorzugsweise Wasser, jeweils bezogen auf das Gewicht des wasserabsorbierenden Polymergebildes, in Kontakt gebracht.

Bei Polymergebilden in der Form von vorzugsweise kugelförmigen Teilchen ist es erfindungsgemäß weiterhin bevorzugt, dass das in Kontakt bringen derart erfolgt, dass lediglich der Aussenbereich, nicht jedoch der innere Bereich der teilchenförmigen Polymergebilde mit dem Fluid $F_1$ und somit dem Nachvernetzer in Kontakt gebracht werden.

Als Nachvernetzer, die im erfindungsgemäßen Verfahren eingesetzt werden, werden vorzugsweise Verbindungen verstanden, die mindestens zwei funktionelle Gruppen aufweisen, die mit funktionellen Gruppen eines Polymergebildes in einer Kondensationsreaktion (=Kondensationsvernetzer), in einer Additionsreaktion oder in einer Ringöffnungsreaktion reagieren können. Als Nachvernetzer sind im erfindungsgemäßen Verfahren diejenigen bevorzugt, die in WO 2004/037903 A2 als Vernetzer der Vernetzerklassen II genannt wurden.

Unter diesen Verbindungen sind als Nachvernetzer besonders bevorzugt Kondensationsvernetzer wie beispielsweise Epoxide, wie etwa Ethylenglykoldiglycidylether oder Dietehylenglykoldiglycidylether, Ethylenglykole, wie etwa Diethylenglykol, Triethylenglykol oder Polyethylenglykol, Glyzerin, Polyglyzerin, Propylenglykole, wie etwa Dipropylenglykol, Tripropylenglykol oder Polypropylenglykol Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on sowie 1,3-Dioxolan-2-on.

Nachdem die Polymergebilde bzw. die Hydrogele mit dem Nachvernetzer bzw. mit dem Fluid $F_1$ beinhaltend den Nachvernetzer in Kontakt gebracht wurden, werden sie auf eine Temperatur im Bereich von 50 bis 300°C, vorzugsweise 75 bis 275°C und besonders bevorzugt 150 bis 250°C erhitzt, so dass, vorzugsweise wodurch, der Aussenbereich der Polymergebilde im Vergleich zum Innenbereich stärker vernetzt wird (=Nachvernetzung). Die Zeitdauer der Wärmebehandlung wird durch die Gefahr, dass das gewünschte Eigenschaftsprofil der Polymergebilde infolge von Hitzeeinwirkung zerstört wird, begrenzt.

Die vorstehend beschriebene Oberflächennachvernetzung kann nicht nur, wie soeben dargelegt, vor dem Verfahrensschritt ii) erfolgen, denkbar ist grundsätzlich auch, die Oberflächennachvernetzung während oder erst nach dem Verfahrensschritt ii) durchzuführen.

In einer bevorzugten Ausführungsform zeigt das im Verfahrensschritt i) des erfindungsgemäßen Verfahren bereitgestellte, unbehandelte wasserabsorbierende Polymergebilde mindestens eine der folgenden Eigenschaften (ERT = *EDANA Recommended Test*):

(A) die maximale Aufnahme gemäß ERT 440.2-02 (im Falle von Partikeln bestimmt für die gesamte Partikelgrößenfraktion) von 0,9 gew.-%iger NaCl-Lösung liegt in einem Bereich von mindestens 10 bis 1000 g/g, vorzugsweise im Bereich von 20 bis 500 g/g und darüber hinaus bevorzugt im Bereich von 50 bis 250 g/g,

(B) der gemäß ERT 470.2-02 (im Falle von Partikeln bestimmt für die gesamte Partikelgrößenfraktion) extrahierbare Anteil nach 16 Stunden beträgt weniger als 30 Gew.-%, vorzugsweise weniger als 20 Gew.-% und darüber hinaus bevorzugt weniger als 15 Gew.-%, jeweils bezogen auf das unbehandelte, wasserabsorbierende Polymergebilde,

(C) die Schüttdichte gemäß ERT 460.2-02 (im Falle von Partikeln bestimmt für die gesamte Partikelgrößenfraktion) liegt im Bereich von 300 bis 1000 g/l, vorzugsweise im Bereich von 400 bis 900 g/l und darüber hinaus bevorzugt 500 bis 800 g/l,

(D) der pH-Wert gemäß ERT 400.2-02 (im Falle von Partikeln bestimmt für die gesamte Partikelgrößenfraktion) von 1 g des unbehandelten, wasserabsorbierenden Polymergebildes in 11 Wasser ist kleiner als 6,5, vorzugsweise kleiner als 6,0, besonders bevorzugt kleiner als 5,5 und am meisten bevorzugt kleiner als 5,2, wobei vorzugsweise ein pH-Wert von 1,0, besonders bevorzugt von 2,0, darüber hinaus bevorzugt von 3,0 und am meisten bevorzugt von 4,5 nicht unterschritten wird,

(E) die nach ERT 442.2-02 (im Falle von Partikeln für die gesamte Partikelfraktion) bestimmte Absorption gegen einen Druck von 50 g/cm$^2$ liegt in einem Bereich von 10 bis 26 g/g, vorzugsweise in einem Bereich von 13 bis 25 g/g und am meisten bevorzugt in einem Bereich von 15 bis 24 g/g;

(F) die nach ERT 441.2-02 (im Falle von Partikeln für die gesamte Partikelfraktion) bestimmte, als CRC bezeichnete Retention liegt in einem Bereich von 20 bis 50 g/g, vorzugsweise in einem Bereich von 25 bis 40 g/g und am meisten bevorzugt in einem Bereich von 27 bis 35 g/g.

Gemäß einer besonderen Ausführungsform des erfindungsgemäßen Verfahrens werden im Verfahrensschritt i) Polymergebilde bereitgestellt, die durch die folgenden Eigenschaften bzw. Eigenschaftskombinationen gekennzeichnet sind: (A), (B), (C), (D), (E), (F), (A)(B), (A)(C), (A)(D), (A)(E), (A)(F), (B)(C), (B)(D), (B)(E), (B)(F), (C)(D), (C)(E), (C)(F), (D)(E), (D)(F), (E)(F) und (A)(B)(C)(D)(E)(F), wobei (D) am meisten bevorzugt ist.

Im Verfahrensschritt ii) des erfindungsgemäßen Verfahrens werden die im Verfahrensschritt i) bereitgestellten unbehandelten, wasserabsorbierenden Polymergebilde mit der sauren Komponente in Kontakt gebracht, wobei es sich bei dieser sauren Komponente um eine organische Säure handelt wie in Anspruch 1 angegeben. Unter die Bezeichnung "saure Komponente" fallen grundsätzlich auch Verbindungen, die erst in Gegenwart von Wasser saure Verbindungen zu bilden vermögen, wie etwa Säureanhydride.

Bevorzugte organische Säuren sind Monocarbonsäuren, Dicarbonsäuren, Tricarbonsäuren, Carbonsäureanhydride oder Mischungen aus mindestens zwei dieser Säuren.

Die vorstehend genannten organischen Säuren sind ausgewählt aus der Gruppe bestehend aus Essigsäureanhydrid, Maleinsäureanhydrid, Fumarsäureanhydrid, Benzoesäure, Ameisensäure, Valeriansäure, Zitronensäure, Glyoxylsäure, Glykolsäure, Glycerinphosphorsäure, Glutarsäure, Chloressigsäure, Chlorpropionsäure, Zimtsäure, Bernsteinsäure, Essigsäure, Weinsäure, Milchsäure, Brenztraubensäure, Fumarsäure, Propionsäure, 3-Hydroxy-propionsäure, Malonsäure, Buttersäure, Isobuttersäure, Imidinoessigsäure, Apfelsäure, Isethionsäure, Methylmaleinsäure, Adipinsäure, Itaconsäure, Crotonsäure, Oxalsäure, Salizylsäure, Gluconsäure, Gallussäure, Sorbinsäure, Gluconsäure und p-Oxybenzoesäure, wobei Zitronensäure und Weinsäure darüber hinaus bevorzugt und Zitronensäure am meisten bevorzugt ist.

Das in Kontakt bringen der sauren Komponente mit dem unbehandelten, wasserabsorbierenden Polymergebilde im Verfahrensschritt ii) des erfindungsgemäßen Verfahrens erfolgt vorzugsweise durch Vermischen der beiden Komponenten, wobei hierzu geeignete Mischaggregate insbesondere der Patterson-Kelley-Mischer, DRAIS-Turbulenzmischer, Lödigemischer, Ruberg-Mischer, Schneckenmischer, Tellermischer und Wirbelschichtmischer oder aber kontinuierlich arbeitende senkrechte Mischer, in denen das Polymergebilde mittels rotierender Messer in schneller Frequenz gemischt wird (Schugi-Mischer), sind.

Weiterhin kann die saure Komponente in Form eines Fluids $F_2$, umfassend ein Lösungsmittel sowie die in diesem Lösungsmittel gelöste oder dispergierte saure Komponente, oder aber in trockener Form als Pulver mit dem unbehandelten, wasserabsorbierenden Polymergebilde in Kontakt gebracht werden, wobei das in Kontakt bringen der sauren Komponente in Form eines Fluids $F_2$ besonders bevorzugt ist. Geeignete Lösungsmittel sind wiederum, neben Wasser, insbesondere mit Wasser mischbare, organische Lösungsmittel wie etwa Methanol, Ethanol, 1-Propanol, 2-Propanol, 1,2-Propandiol, 1,3-Propandiol, 1-Butanol, 2-Butanol, tert.-Butanol, iso-Butanol oder aber Mischungen aus organischen Lösungsmitteln oder Mischungen aus Wasser mit einem oder mehreren dieser organischen Lösungsmittel, wobei Wasser als Lösungsmittel am meisten bevorzugt ist. Wird das unbehandelte, wasserabsorbierende Polymergebilde mit dem Fluid $F_2$ umfassend das Lösungsmittel und die saure Komponente in Kontakt gebracht, so ist es weiterhin bevorzugt, dass dieses Fluid $F_2$ die saure Komponente in einer Menge in einem Bereich von 0,1 bis 75 Gew.-%, besonders bevorzugt 20 bis 65 Gew.-% und am meisten bevorzugt 30 bis 60 Gew.-%, jeweils bezogen auf das Gesamtgewicht des Fluids $F_2$, beinhaltet.

Gemäß einer besonders bevorzugten Ausführungsform des erfindungsgemäßen Verfahrens wird das unbehandelte, wasserabsorbierende Polymergebildes im Verfahrensschritt ii) mit höchstens 20 Gew.-%, besonders bevorzugt höchstens 15 Gew.-%, noch mehr bevorzugt höchstens 10 Gew.-% und darüber hinaus bevorzugt höchstens 5 Gew.-%, jedoch vorzugsweise mit mindestens 1 Gew.-%, besonders bevorzugt mit mindestens 2 Gew.-%, darüber hinaus bevorzugt mit mindestens 3 Gew.-% und am meisten bevorzugt mindestens 4 Gew.-% eines Lösungsmittels, vorzugsweise Wasser, in Kontakt gebracht, wobei die vorstehend genannten Gew.-%-Angaben auf das Gewicht des unbehandelten, wasserabsorbierenden Polymergebildes bezogen sind.

Weiterhin ist es erfindungsgemäß bevorzugt, dass das unbehandelte, wasserabsorbierende Polymergebilde im Verfahrensschritt ii) mit 0,1 bis 20 Gew.-%, besonders bevorzugt 0,5 bis 15 Gew.-%, darüber hinaus bevorzugt 1 bis 10 Gew.-% und am meisten bevorzugt 2,5 bis 7,5 Gew.-% der sauren Komponente, jeweils bezogen auf das Gewicht des im Verfahrensschritt i) bereitgestellten, unbehandelten, wasserabsorbierenden Polymergebildes, in Kontakt gebracht wird.

Weiterhin kann es vorteilhaft sein, dass das in Kontakt bringen des unbehandelten, wasserabsorbierenden Polymergebilde mit der sauren Komponente im Verfahrensschritt ii) bei einer Temperatur in einem Bereich von 30 bis 210°C, besonders bevorzugt von 40 bis 150°C und am meisten bevorzugt in einem Bereich von 50 bis 100°C erfolgt. Denkbar ist auch, das unbehandelte, wasserabsorbierende Polymergebilde bei einer geringeren Temperatur, beispielsweise bei Raumtemperatur, mit der sauren Komponente in Kontakt zu bringen und die so erhaltene Mischung erst dann auf die vorstehend genannten Temperaturen zu erhitzen.

Das unbehandelte, wasserabsorbierende Polymergebilde wird im Verfahrensschritt ii) zusätzlich zur sauren, vorzugsweise organischen Komponente auch noch mit einer von der sauren Komponente verschiedenen anorganischen Komponente in Kontakt gebracht. Bei dieser anorganischen Komponente handelt es sich um eine Silizium und Sauerstoff beinhaltende anorganische Komponente wie in Anspruch 1 definiert, welche gemäß einer besonderen Ausgestaltung des erfindungsgemäßen Verfahrens als Pulver vorliegt.

Zu den Silizium und Sauerstoff beinhaltenden, anorganischen Komponenten gehören Verbindungen, die durch Polykondensation der Mono-Orthokieselsäure erhältlich sind, sowie Silikate. Unter den Polykieselsäuren besonders bevorzugt sind Kieselsäuresole, wie sie in der DE 102 49 821, beschrieben werden. Unter den Silikaten sind insbesondere Gerüstsilikate wie Zeolithe oder Silikate, die durch Trocknung wässriger Kieselsäurelösungen oder Kieselsäuresolen erhalten wurden, beispielsweise die kommerziell erhältlichen, unter der Bezeichnung Aersoil® bekannten pyrogenen Kieselsäuren, welche vorzugsweise ein Teilchengröße im Bereich von 5 bis 50 nm, besonders bevorzugt im Bereich von 8 bis 20 nm aufweisen, bevorzugt. Auch Fällungskieselsäuren, insbesondere die unter der Bezeichnung Sipernat® bekannten Fällungskieselsäuren kommen in Betracht. Bevorzugte Silikate sind weiterhin alle natürlichen oder synthetischen Silikate, die in "Holleman und Wiberg, Lehrbuch der Anorganischen Chemie, Walter de Gruyter-Verlag, 91.-100. Auflage, 1985" auf den Seiten 750 bis 783, als Silikate offenbart sind.

Besonders bevorzugte Zeolithe sind natürliche Zeolithe aus der Natrolith-Gruppe, der Harmotom-Gruppe, der Mordenit-Gruppe, der Chabasit-Gruppe, der Faujasit-Gruppe (Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabazit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmotom, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS.

Unter den Silizium und Sauerstoff beinhaltenden, anorganischen Komponenten bevorzugt ist jedoch pyrogene Kieselsäure, wie sie beispielsweise unter der Handelbezeichnung Aerosil® erhältlich ist, Kieselsäuresol, wie es beispielsweise unter der Handelsbezeichnung Levasil® erhältlich ist oder Fällungskieselsäure, wie sie beispielsweise unter der Handelsbezeichnung Sipernat® erhältlich ist.

Weiterhin wird das unbehandelte, wasserabsorbierende Polymergebilde im Verfahrensschritt ii) mit 0,001 bis 5 Gew.-%, besonders bevorzugt 0,01 bis 2,5 Gew.-% und am meisten bevorzugt 0,1 bis 1 Gew.-% der anorganischen Komponente, jeweils bezogen auf das Gewicht des im Verfahrensschritt i) bereitgestellten, unbehandelten, wasserabsorbierenden Polymergebildes, in Kontakt gebracht.

Es sind verschiedene Möglichkeiten des in Kontakt bringens denkbar:

- gemäß einer ersten Variante wird das unbehandelte, gegebenenfalls bereits oberflächennachvernetzte, wasserabsorbierende Polymergebilde zunächst mit der sauren Komponente, entweder in Pulverform oder in Form des Fluids $F_2$, vorzugsweise in Form des Fluids $F_2$, in Kontakt gebracht und anschließend wird die so erhaltene Mischung mit der anorganischen, vorzugsweise pulverförmigen Komponente in Kontakt gebracht;

- gemäß einer zweiten und besonders bevorzugten Variante wird das unbehandelte, gegebenenfalls bereits oberflächennachvernetzte, wasserabsorbierende Polymergebilde zunächst mit der vorzugsweise pulverförmigen, anorganischen Komponente in Kontakt und anschließend wird die so erhaltene Mischung mit der sauren Komponente, entweder in Pulverform oder in Form des Fluids $F_2$, vorzugsweise in Form des Fluids $F_2$, in Kontakt gebracht;

- gemäß einer dritten Variante wird das unbehandelte, gegebenenfalls bereits oberflächennachvernetzte, wasserabsorbierende Polymergebilde zeitgleich mit der vorzugsweise pulverförmigen, anorganischen Komponente und der sauren Komponente, entweder in Pulverform oder in Form des Fluids $F_2$, vorzugsweise in Form des Fluids $F_2$, in Kontakt gebracht. Hierbei könnten die vorzugsweise pulverförmige, anorganische Komponente und die vorzugsweise in Form des Fluids $F_2$ vorliegende saure Komponente getrennt zum unbehandelten, wasserabsorbierenden Polymergebilde gegeben werden. Denkbar ist jedoch grundsätzlich auch, dass zunächst die vorzugsweise pulverförmige, anorganische Komponente mit der vorzugsweise in Form des Fluids $F_2$ vorliegenden sauren Komponente vermischt und anschließend die so erhaltene Mischung mit dem unbehandelten, wasserabsorbierenden Polymergebilde in Kontakt gebracht wird.

Insbesondere dann, wenn im erfindungsgemäßen Verfahren im Verfahrensschritt ii) die wasserabsorbierenden Polymergebilde nicht nur mit der saueren, sondern zusätzlich auch mit der anorganischen Komponente in Kontakt gebracht werden, ist es bevorzugt, dass die saure Komponente in Form des vorstehend beschriebenen Fluids $F_2$ eingesetzt wird, wobei in diesem Fall die wasserabsorbierenden Polymergebilde mit mindestens 1 Gew.-%, besonders bevorzugt mit mindestens 2 Gew.-%, darüber hinaus bevorzugt mit mindestens 3 Gew.-% und am meisten bevorzugt mindestens 4 Gew.-% des Lösungsmittels, in dem sie saure Komponente gelöst oder dispergiert ist, in Kontakt gebracht werden.

An den Verfahrenschritt ii) des erfindungsgemäßen Verfahrens kann sich auch noch ein Verfahrensschritt

iii) weitere Oberflächenmodifizierung des im Verfahrensschritt ii) erhaltenen, wasserabsorbierenden Polymergebildes,

anschließen.

Als weitere Oberflächenmodifizierung denkbar ist weiterhin das in Kontakt bringen der im Verfahrensschritt ii) erhaltenen, wasserabsorbierenden Polymergebilden mit permeabilitätssteigernden Mitteln, beispielsweise mit Salzen. Bevorzugte Salze sind Phosphate oder aber Salze umfassend ein mehrwertiges, vorzugsweise dreiwertiges Kation. Unter diesen Salzen besonders bevorzugt sind Salze beinhaltend Chlorid-Anionen, Iodid-Anionen, Bromid-Anionen, Nitrat-Anionen, Nitrit-Anionen, Sulfid-Anionen, Sulfit-Anionen, Sulfat-Anionen, Carbonat-Anionen, Hydrogencarbonat-Anionen, Hydroxid-Anionen oder organische Anionen wie Acetat-Anionen oder Oxalat-Anionen. Besonders bevorzugte Salze umfassend ein dreiwertiges Kation sind Aluminiumchlorid, Polyaluminiumchlorid, Aluminiumsulfat, Aluminiumnitrat, bis-Aluminium-Kalium-Sulfat, bis-Aluminium-Natrium-Sulfat, Aluminiumlactat, Aluminiumoxalat, Aluminiumcitrat, Aluminiumglyoxylat, Aluminiumsuccinat, Aluminiumitaconat, Aluminiumbutyrat, Aluminiumsorbat, Aluminiummalonat, Aluminiumbenzoat, Aluminiumcrotonat, Aluminiumtartrat, Aluminiumpyruvat, Aluiniumvalerat, Aluminiumformat, Aluminiumglutarat, Aluminiumpropanoat und Aluminiumacetat, wobei $AlCl_3 \times 6H_2O$, $NaAl(S0_4)_2 \times 12\ H_2O$, $Al(NO_3)_3 \times 9\ H_2O$, $KAl(S0_4)_2 \times 12\ H_2O$ oder $Al_2(SO_4)_3 \times 14\text{-}18\ H_2O$ sowie die entsprechenden wasserfreien Salze, $Na_2SO_4$ oder dessen Hydrate, $MgSO_4 \times 10\ H_2O$ oder wasserfreies Magnesiumsulfat am meisten bevorzugt sind.

Weiterhin kann es sich bei dieser weiteren Oberflächenmodifizierung um ein in Kontakt bringen des im Verfahrensschritt ii) erhaltenen, wasserabsorbierenden Polymergebildes mit einer Verbindung, welche die Staubbildung vermindert, wie etwa einem Polyvinylalkohol, oder mit einer Verbindung, welche in der Lage ist, Gerüche zu binden, wie etwa einem Cyclodextrin oder einem Zeolithen, handeln.

Einen weiteren Beitrag zur Lösung der eingangs genannten Aufgaben leistet ein wasserabsorbierendes Polymergebilde, welches erhältlich ist durch das vorstehend beschriebene Verfahren. Dieses wasserabsorbierende Polymergebilde umfasst einen Innenbereich und einen den Innenbereich umgebenden Außenbereich, wobei der Außenbereich der wasserabsorbierenden Polymergebilde mit der vorstehend beschriebenen, sauren Komponente und mit der vorstehend beschriebenen anorganischen, pulverförmigen Komponente in Kontakt gebracht worden ist. Dabei ist das durch das erfindungsgemäße Verfahren erhältliche, wasserabsorbierende Polymergebilde insbesondere dadurch gekennzeichnet, dass es hinsichtlich seines Neutralisationsgrades inhomogen ist. "Inhomogen hinsichtlich seines Neutralisationsgrades" bedeutet dabei, das der Außenbereich des wasserabsorbierenden Polymergebildes einen geringeren Neutralisationsgrad, vorzugsweise einen um mindestens 1%, besonders bevorzugt um mindestens 2,5 %, noch mehr bevorzugt um mindestens 5 % und am meisten bevorzugt um mindestens 10 % geringeren Neutralisationsgrad aufweist als der Innenbereich. Weist beispielsweise der Innenbereich einen Neutralisationsgrad von 60 Mol-% auf, so beträgt der Neutralisationsgrad im Außenbereich vorzugsweise höchstens 59 Mol-%, besonders bevorzugt höchstens 57,5 Mol-%, noch mehr bevorzugt höchstens 55 Mol-% und am meisten bevorzugt höchstens 50 Mol-%.

Das durch das vorstehend beschriebene Verfahren erhältliche wasserabsorbierende Polymergebilde weist mindestens eine, vorzugsweise alle, der folgenden Eigenschaften auf:

($\beta$1) eine nach ERT 441.2-02 für die gesamte Partikelfraktion bestimmte Retention von mindestens 27 g/g, besonders bevorzugt mindestens 29 g/g, darüber hinaus bevorzugt mindestens 31 g/g und am meisten bevorzugt mindestens 33 g/g, wobei vorzugsweise eine Retention von 50 g/g, besonders bevorzugt 45 g/g und am meisten bevorzugt 40 g/g nicht überschritten wird;

($\beta$2) einen SAP-Index von mindestens 140 cm$^3$s/g, vorzugsweise von mindestens 160 cm$^3$s/g, darüber hinaus bevorzugt von mindestens 180 cm$^3$s/g und am meisten bevorzugt von mindestens 200 cm$^3$s/g, wobei der SAP-Index wie folgt definiert ist

$$\text{SAP-Index} = (\text{RET} \times \text{SFC})/\text{pH}$$

und wobei

RET  = die gemäß ERT 441.2-02 für die gesamte Partikelfraktion bestimmte Retention,

SFC  = die gemäß der hierin beschriebenen Testmethode für die gesamte Partikelfraktion bestimmte Permeabilität und

pH  = der gemäß ERT 400.2-02 für die gesamte Partikelfraktion bestimmte pH-Wert ist;

(β3) eine nach ERT 442.2.02 bestimmte Absorption unter einem Druck von 50 g/cm$^2$ von höchstens 20 g/g, besonders bevorzugt höchstens 19 g/g und am meisten bevorzugt höchstens 18 g/g, wobei vorzugsweise eine Absorption unter einem Druck von 50 g/cm$^2$ von 5 g/g, besonders bevorzugt 10 g/g und am meisten bevorzugt 15 g/g nicht unterschritten wird;

(β4) ein gemäß der hierin beschriebenen Testmethode bestimmtes Ammoniak-Bindevermögen von mindestens 98 mg/g, besonders bevorzugt von mindestens 99 mg/g und am meisten bevorzugt von mindestens 100 mg/g, wobei vorzugsweise ein Ammoniak-Bindevermögen von 130 mg/g, besonders bevorzugt 120 mg/g und am meisten bevorzugt 110 mg/g nicht überschritten wird;

(β5) ein gemäß ERT 400.2-02 (im Falle von Partikeln bestimmt für die gesamte Partikelgrößenfraktion) bestimmter pH-Wert von weniger als 6,5, vorzugsweise weniger als 6,0, besonders bevorzugt weniger als 5,5 und am meisten bevorzugt weniger als 5, wobei vorzugsweise ein pH-Wert von 1,0, besonders bevorzugt von 2,0, darüber hinaus bevorzugt von 3,0 und am meisten bevorzugt von 4,0 nicht unterschritten wird.

Bevorzugte, durch das erfindungsgemäße Verfahren erhältliche wasserabsorbierende Polymergebilde sind diejenigen, die durch folgende Eigenschaften oder Kombination von Eigenschaften gekennzeichnet sind: (β1), (β2), (β1)(β2), (β1)(β2)(β3), (β1)(β2)(β4), (β1)(β2)(β5), und (β1)(β2)(β3)(β4)-(β5), wobei die Kombination (β1)(β2)(β5), insbesondere mit einer Retention von mindestens 27 g/g und einem pH-Wert weniger als 5,5, am meisten bevorzugt ist.

Einen Beitrag zur Lösung der eingangs genannten Aufgabe leistet auch ein wasserabsorbierendes Polymergebilde umfassend einen Innenbereich und einen den Innenbereich umgebenden Außenbereich, wobei der Außenbereich des wasserabsorbierenden Polymergebildes mit der vorstehend beschriebenen, sauren Komponente und gegebenenfalls auch mit der vorstehend beschriebenen anorganischen, vorzugsweise pulverförmigen Komponente in Kontakt gebracht worden ist und wobei das wasserabsorbierende Polymergebilde mindestens eine, vorzugsweise alle, der folgenden Eigenschaften aufweist:

(β1) eine nach ERT 441.2-02 bestimmte Retention von mindestens 27 g/g, besonders bevorzugt mindestens 29 g/g, darüber hinaus bevorzugt mindestens 31 g/g und am meisten bevorzugt mindestens 33 g/g, wobei vorzugsweise eine Retention von 50 g/g, besonders bevorzugt 45 g/g und am meisten bevorzugt 40 g/g nicht überschritten wird;

(β2) einen SAP-Index (SAPI) von mindestens 140 cm$^3$s/g, vorzugsweise von mindestens 160 cm$^3$s/g, darüber hinaus bevorzugt von mindestens 180 cm$^3$s/g und am meisten bevorzugt von mindestens 200 cm$^3$s/g, wobei der SAP-Index wie folgt definiert ist

$$\text{SAP-Index} = (\text{RET} \times \text{SFC})/\text{pH}$$

und wobei

RET  = die gemäß ERT 441.2-02 für die gesamte Partikelfraktion bestimmte Retention,

SFC  = die gemäß der hierin beschriebenen Testmethode für die gesamte Partikelfraktion bestimmte Permeabilität und

pH  = der gemäß ERT 400.2-02 für die gesamte Partikelfraktion bestimmte pH-Wert ist;

(β3) eine nach ERT 442.2.02 bestimmte Absorption unter einem Druck von 50 g/cm$^2$ von höchstens 20 g/g, besonders bevorzugt höchstens 19 g/g und am meisten bevorzugt höchstens 18 g/g, wobei vorzugsweise eine Absorption unter einem Druck von 50 g/cm$^2$ von 5 g/g, besonders bevorzugt 10 g/g und am meisten bevorzugt 15 g/g nicht unterschritten wird;

(β4) ein gemäß der hierin beschriebenen Testmethode bestimmtes Ammoniak-Bindevermögen von mindestens 98 mg/g, besonders bevorzugt von mindestens 99 mg/g und am meisten bevorzugt von mindestens 100 mg/g, wobei vorzugsweise ein Ammoniak-Bindevermögen von 130 mg/g, besonders bevorzugt 120 mg/g und am meisten bevorzugt 110 mg/g nicht überschritten wird;

(β5) ein gemäß ERT 400.2-02 (im Falle von Partikeln bestimmt für die gesamte Partikelgrößenfraktion) bestimmter pH-Wert von weniger als 6,5, vorzugsweise weniger als 6,0, besonders bevorzugt weniger als 5,5 und am meisten bevorzugt weniger als 5, wobei vorzugsweise ein pH-Wert von 1,0, besonders bevorzugt von 2,0, darüber hinaus bevorzugt von 3,0 und am meisten bevorzugt von 4,0 nicht unterschritten wird.

Bevorzugte erfindungsgemäße wasserabsorbierende Polymergebilde sind diejenigen, die durch folgende Eigenschaften oder Kombination von Eigenschaften gekennzeichnet sind: (β1), (β2), (β1)(β2), (β1)(β2)(β3), (β1)(β2)(β3), (β1)(β2)(β4), (β1)(β2)(β5), und (β1)(β2)(β3)(β4)(β5), wobei die Kombination (β1)(β2)(β5), insbesondere mit einer Retention von mindestens 27 g/g und einem pH-Wert weniger als 5,5, am meisten bevorzugt ist.

Einen weiteren zur Lösung der eingangs beschriebenen Aufgaben liefert ein Verbund, beinhaltend die erfindungsgemäßen wasserabsorbierenden Polymergebilde bzw. die durch das erfindungsgemäße Verfahren erhältlichen wasserabsorbierenden Polymergebilde (nachfolgend als - erfindungsgemäße wasserabsorbierende Polymergebilde - genannt) und ein Substrat. Es ist dabei bevorzugt, dass die erfindungsgemäßen Polymergebilde und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich umfasst, welcher das erfindungsgemäße wasserabsorbierende Polymergebilde in einer Menge im Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm$^3$, vorzugsweise mindestens 0,1 cm$^3$ und am meisten bevorzugt mindestens 0,5 cm$^3$ aufweist.

In einer besonders bevorzugten Ausführungsform des erfindungsgemäßen Verbundes handelt es sich um einen flächenförmigen Verbund, wie er in der WO 02/056812 A1 als "absorbent material" beschrieben ist, insbesondere hinsichtlich des genauen Aufbaus des Verbundes, des Flächengewichtes seiner Bestandteile sowie seiner Dicke.

Einen weiteren Beitrag zur Lösung der Eingangs genannten Aufgaben liefert ein Verfahren zur Herstellung eines Verbundes, wobei die erfindungsgemäßen wasserabsorbierenden Polymergebilde und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Einen Beitrag zur Lösung der Eingangs genannten Aufgaben liefert auch ein Verbund erhältlich nach dem vorstehend beschriebenen Verfahren, wobei dieser Verbund vorzugsweise die gleichen Eigenschaften aufweist wie der vorstehend beschriebene, erfindungsgemäße Verbund.

Einen weiteren Beitrag zur Lösung der Eingangs genannten Aufgaben liefern chemische Produkte beinhaltend die erfindungsgemäßen Polymergebilde oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen Polymergebilde oder des erfindungsgemäßen Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierende Mittel oder Pflanzenschutzwirkstoffe liefern einen Beitrag zur Lösung der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Die Erfindung wird nun anhand von Testmethoden und Beispielen näher erläutert.

TESTMETHODEN

Bestimmung des SFC-Wertes

Die Bestimmung des SFC-Wertes erfolgte gemäß dem in der WO 95/26209 A1 beschriebenen Testverfahren.

Bestimmung des Ammoniak-Bindevermögens

85 ml einer 0,9 gew.-%igen Natriumchloridlösung werden in einem mit einem Glasstopfen verschließbaren 200 ml Erlenmeyerkolben vorgelegt und mittels eines Magnetrührers gerührt. Zu dieser Lösung werden 15 ml einer 0,1 molaren NaOH-Lösung, die frei von Carbonaten ist, mittels einer Bürette zugesetzt. Dann werden etwa 200 mg des zu analysierenden Superabsorbers exakt abgewogen und in die im Erlenmeyerkolben befindliche Lösung eingestreut. Der Erlenmeyerkolben wird mittels eines Glasstopfens verschlossen und die so erhaltene Zusammensetzung wird für 60 Minuten bei 500 Umdrehungen pro Minute gerührt.

Anschließend wird die Zusammensetzung mittels eines Filterpapiers (Schwarzband von Schleicher & Schull) filtriert und 50 ml des so erhaltenen Filtrates werden anschließend mittels einer 0,1 molaren HCl-Lösung auf einem Titroprocessor (Metrohm 670 der Firma Metrohm GmbH & Co) bis zum ersten Endpunkt titriert. Als Kontrollwert dient eine entsprechende Lösung (85 ml 0,9 gew.-%ige NaCl-Lösung + 15 ml 0,1 molare NaOH-Lösung) ohne Superabsorber.

Die Ammoniak-Bindekapazität w wird wie folgt ermittelt:

$$w\,[mg\,/\,g] = \frac{(V_1 - V_2) \times c \times F \times M}{m}$$

wobei

w     die Ammoniak-Bindekapazität,
$V_1$     der Verbrauch an HCl-Lösung in ml für den Kontrollwert,
$V_2$     der Verbrauch an HCl-Lösung in ml für die Lösung mit dem Superabsorber,
c     die Konzentration der HCl-Lösung (0,1 mol/l),
M     die Molare Masse des Ammoniaks (17,03 g/mol),
F     der Faktor 2, berechnet aus dem Verhältnis 100 ml/50 ml und
m     die Menge an eingesetztem Superabsorber in g

ist.

Es werden jeweils 6-fach Bestimmungen durchgeführt und die Ammoniak-Bindekapazität als Mittelwert dieser Bestimmungen angegeben.

BEISPIELE

1. Herstellung von unbehandelten, wasserabsorbierenden Polymergebilden (Verfahrensschritt i)

Eine Monomerlösung bestehend aus 2.400 g Acrylsäure, 1.332,2 g NaOH (50%ig), 4.057,4 g entionisiertes Wasser, 2,14 g Polyethylenglykol-300-diacrylat (mit einem Gehalt an wirksamer Substanz von 78,4 Gew.-%), 6,92 g Monoallylpolyethylenglykol-450-monoacrylsäureester (mit einem Gehalt an wirksamer Substanz von 72,8 Gew.-%) und 65,36 g Polyethylenglykol-750-monomethacrylsäure-estermethylether (mit einem Gehalt an wirksamer Substanz von 73,4 Gew.-%) wurde durch Spülen mit Stickstoff vom gelösten Sauerstoff befreit und auf die Starttemperatur von 4°C abgekühlt. Nach Erreichen der Starttemperatur wurde die Initiatorlösung (2,4 g Natriumperoxydisulfat in 77,6 g $H_2O$, 0,56 g 30%ge WasserstoffperoxidLösung in 15,44 g $H_2O$ und 0,12 g Ascorbinsäure in 39,88 g $H_2O$) zugesetzt. Nachdem die Endtemperatur von ca. 100°C erreicht war, wurde das entstandene Gel mit einem Fleischwolf zerkleinert und bei 150°C 2 Stunden lang im Trockenschrank getrocknet. Das getrocknete Polymerisat wurde grob zerstoßen, mittels einer Schneidmühle SM 10 mit einem 2 mm-Sieb gemahlen und auf ein Pulver mit einer Partikelgröße von 150 bis 850 $\mu$m gesiebt (=Pulver A).

2. Oberflächennachvernetzung (noch Verfahrensschritt i)

Das Pulver A wurde mit einer wässrigen Lösung bestehend aus Ethylencarbonat (1 Gew.-% bezogen auf das Pulver A) und Wasser (3 Gew.-% bezogen auf das Pulver A) in einem Labormischer vermischt und anschließend im Ofen für eine Zeitdauer von 30 Minuten auf 150°C erhitzt (=Pulver B)

3. Erfindungsgemäße Beschichtung der Pulver

Das Pulver B wurde mit den in der nachfolgenden Tabelle angegebenen Mengen an trockenem Aerosil®200, den in der Tabelle angegebenen Mengen an Zitronensäure und den in der Tabelle angegebenen Mengen an Wasser als Lösungsmittel versetzt (alle Gew.-%-Angaben sind auf das wasserabsorbierende Polymergebilde bezogen). Die Zitronensäure wurde in Form einer 50 Gew.-%igen Zitronensäurelösung mittels einer Spritze und einer 0,9 mm-Kanüle bei 750 Umdrehungen pro Minute zum Pulver B zugesetzt. Wurde sowohl Aerosil®200 als auch Zitronensäurelösung zugesetzt, so wurde zunächst das Aerosil®200 trocken in das Pulver B eingerührt und danach auf einer Rollbank für 30 Minuten homogenisiert und anschließend wurde die Zitronensäurelösung, wie vorstehend beschrieben, mittels einer Spritze zugesetzt.

| | Pulver B (n. E.[1]) | Pulver C (n. E.[1]) | Pulver D (n .E.[1]) | Pulver E (E.[2]) | Pulver F (E.[2]) |
|---|---|---|---|---|---|
| Aerosil®200 | 0 | 0,5 | 0 | 0,5 | 1,0 |
| Zitronensäure | 0 | 0 | 5,0 | 5,0 | 5,0 |
| Wasser | 0 | 5,0 | 5,0 | 5,0 | 5,0 |
| pH-Wert | 5,30 | 5,36 | 5,11 | 5,15 | 4,93 |
| Retention [g/g] | 29,8 | 30,8 | 29,0 | 29,0 | 29,7 |
| SAP-Index | 5,6 | 40,2 | 141,0 | 180,0 | 174,0 |
| Ammoniak-Bindevermögen [mg/g] | 95,1 | 83,3 | 100,3 | 100,5 | 99,7 |
| [1] n. E. = nicht erfindungsgemäß [2] E. = erfindungsgemäß | | | | | |

## Patentansprüche

1. Verfahren zur Herstellung eines wasserabsorbierenden Polymergebildes, umfassend die Verfahrensschritte:

    i) Bereitstellen eines unbehandelten, wasserabsorbierenden Polymergebildes mit einem Neutralisationsgrad von höchstens 70 Mol-%;
    ii) In Kontakt bringen des unbehandelten, wasserabsorbierenden Polymergebildes mit einer organischen Säure ausgewählt aus der Gruppe bestehend aus Anissäure, Benzoesäure, Ameisensäure, Valeriansäure, Zitronensäure, Glyoxylsäure, Glykolsäure, Glycerinphos- phorsäure, Glutarsäure, Chloressigsäure, Chlorpropionsäure, Zimtsäure, Bernsteinsäure, Essigsäure, Weinsäure, Milchsäure, Brenztraubensäure, Fumarsäure, Propionsäure, 3-Hydroxypropionsäure, Malonsäure, Buttersäure, Isobuttersäure, Imidinoessigsäure, Apfelsäure, Isethionsäure, Methylmaleinsäure, Adipinsäure, Itaconsäure, Crotonsäure, Oxalsäure, Salizylsäure, Gallussäure, Sorbinsäure, Gluconsäure und p-Oxybenzoesäure als sauren Komponente,

    wobei das im Verfahrensschritt i) bereitgestellte, unbehandelte, wasserabsorbierende Polymergebilde oberflächennachvernetzt ist,
    und wobei das wasserabsorbierende Polymergebilde im Verfahrensschritt ii) zusätzlich mit 0,001 bis 5 Gew.-% einer anorganischen Komponente, bei der es sich um pyrogene Kieselsäure, Kieselsäuresol oder Fällungskieselsäure handelt, in Kontakt gebracht wird, bezogen auf das Gewicht des im Verfahrensschritt i) bereitgestellten, unbehandelten, wasserabsorbierenden Polymergebildes.

2. Verfahren nach Anspruch 1, wobei der Neutralisationsgrad des im Verfahrensschritt i) bereitgestellten, unbehandelten, wasserabsorbierenden Polymergebildes höchstens 60 Mol-% beträgt.

3. Verfahren nach Anspruch 1 oder 2, wobei der Neutralisationsgrad des im Verfahrensschritt i) bereitgestellten, unbehandelten, wasserabsorbierenden Polymergebildes höchstens 55 Mol-% beträgt.

4. Verfahren nach einem der vorhergehenden Ansprüche, wobei das unbehandelte, wasserabsorbierende Polymergebilde im Verfahrensschritt ii) mit einem Fluid $F_2$ beinhaltend die saure Komponente in Kontakt gebracht wird.

5. Verfahren nach einem der vorhergehenden Ansprüche, wobei das unbehandelte, wasserabsorbierende Polymergebilde im Verfahrensschritt ii) mit 0,1 bis 20 Gew.-% der sauren Komponente, bezogen auf das Gewicht des im Verfahrensschritt i) bereitgestellten, unbehandelten, wasserabsorbierenden Polymergebildes, in Kontakt gebracht wird.

6. Wasserabsorbierendes Polymergebilde, erhältlich durch ein Verfahren nach einem der Ansprüche 1 bis 5, umfassend einen Innenbereich und einen den Innenbereich umgebenden Aussenbereich, wobei der Aussenbereich der wasserabsorbierenden Polymergebilde mit einer organischen Säure ausgewählt aus der Gruppe bestehend aus Anissäure, Benzoesäure, Ameisensäure, Valeriansäure, Zitronensäure, Glyoxylsäure, Glykolsäure, Glycerinphosphorsäure, Glutarsäure, Chloressigsäure, Chlorpropionsäure, Zimtsäure, Bernsteinsäure, Essigsäure, Weinsäure, Milchsäure, Brenztraubensäure, Fumarsäure, Propionsäure, 3-Hydroxypropionsäure, Malonsäure, Buttersäure, Isobuttersäure, Imidinoessigsäure, Apfelsäure, Isethionsäure, Methylmaleinsäure, Adipinsäure, Itaconsäure, Crotonsäure, Oxalsäure, Salizylsäure, Gallussäure, Sorbinsäure, Gluconsäure und p-Oxybenzoesäure als sauren Komponente und mit einer pulverförmigen Komponente, bei der es sich um pyrogene Kieselsäure, Kieselsäuresol oder Fällungskieselsäure handelt, in Kontakt gebracht worden ist, und wobei das wasserabsorbierende Polymergebilde mindestens eine der folgenden Eigenschaften aufweist:

(ß1) eine nach ERT 441.2-02 bestimmte Retention von mindestens 27 g/g;
(ß2) einen SAP-Index (SAPI) von mindestens 140 cm$^3$ s/g, wobei der SAP- Index wie folgt definiert ist

$$SAP\text{--}Index = (RET \times SFC)/pH$$

und wobei

RET = die gemäß ERT 441.2-02 bestimmte Retention,
SFC = die gemäß der hierin beschriebenen Testmethode bestimmte Permeabilität und pH = der gemäß ERT 400.2-02 bestimmte pH- Wert ist;

(ß3) eine nach ERT 442.2.02 bestimmte Absorption unter einem Druck von 50 g/cm$^2$ von höchstens 20 g/g;
(ß4) ein gemäß der hierin beschriebenen Testmethode bestimmtes Ammoniak-Bindevermögen von mindestens 98 mg/g;
(ß5) ein gemäß ERT 400.2-02 bestimmter pH- Wert von weniger als 6,5.

7. Schäume, Formkörper, Fasern, Folien, Filme, Kabel, Dichtungsmaterialien, flüssigkeitsaufnehmende Hygieneartikel, Träger für pflanzen- und pilzwachstumregulierende Mittel, Verpackungsmaterialien, Bodenzusätze oder Baustoffe, beinhaltend das wasserabsorbierende Polymergebilde nach Anspruch 6.

8. Verwendung des wasserabsorbierenden Polymergebildes nach Anspruch 6 in Schäumen, Formkörpern, Fasern, Folien, Filmen, Kabeln, Dichtungsmaterialien, flüssigkeitsaufnehmenden Hygieneartikeln, Trägern für pflanzen- und pilzwachstumregulierenden Mitteln, Verpackungsmaterialien, Bodenzusätzen, zur kontrollierten Freisetzung von Wirkstoffen oder in Baustoffen.

**Claims**

1. Process for the preparation of a water-absorbing polymer structure, comprising the process steps:

i) providing an untreated water-absorbing polymer structure having a degree of neutralization of at most 70 mol%;
ii) bringing the untreated water-absorbing polymer structure into contact with an organic acid chosen from the group consisting of anisic acid, benzoic acid, formic acid, valeric acid, citric acid, glyoxylic acid, glycollic acid, glycerol phosphoric acid, glutaric acid, chloroacetic acid, chloropropionic acid, cinnamic acid, succinic acid, acetic acid, tartaric acid, lactic acid, pyruvic acid, fumaric acid, propionic acid, 3-hydroxypropionic acid, malonic acid, butyric acid, isobutyric acid, imidinoacetic acid, malic acid, isethionic acid, methylmaleic acid, adipic acid, itaconic acid, crotonic acid, oxalic acid, salicylic acid, gallic acid, sorbic acid, gluconic acid and p-oxybenzoic acid as acidic component,

wherein the untreated water-absorbing polymer structure provided in process step i) is post-crosslinked on the surface,

and wherein the water-absorbing polymer structure is additionally brought into contact with 0.001 to 5 wt.% of an inorganic component which is pyrogenic silica, silica sol or precipitated silica in process step ii), based on the weight of the untreated water-absorbing polymer structure provided in process step i).

2. Process according to Claim 1, wherein the degree of neutralization of the untreated water-absorbing polymer structure provided in process step i) is at most 60 mol%.

3. Process according to Claim 1 or 2, wherein the degree of neutralization of the untreated water-absorbing polymer structure provided in process step i) is at most 55 mol%.

4. Process according to one of the preceding claims, wherein in process step ii) the untreated water-absorbing polymer structure is brought into contact with a fluid $F_2$ comprising the acidic component.

5. Process according to one of the preceding claims, wherein in process step ii) the untreated water-absorbing polymer structure is brought into contact with 0.1 to 20 wt.% of the acidic component, based on the weight of the untreated water-absorbing polymer structure provided in process step i).

6. Water-absorbing polymer structure obtainable by a process according to one of Claims 1 to 5, comprising an inner region and an outer region surrounding the inner region, wherein the outer region of the water-absorbing polymer structure has been brought into contact with an organic acid chosen from the group consisting of anisic acid, benzoic acid, formic acid, valeric acid, citric acid, glyoxylic acid, glycollic acid, glycerol phosphoric acid, glutaric acid, chloroacetic acid, chloropropionic acid, cinnamic acid, succinic acid, acetic acid, tartaric acid, lactic acid, pyruvic acid, fumaric acid, propionic acid, 3-hydroxypropionic acid, malonic acid, butyric acid, isobutyric acid, imidinoacetic acid, malic acid, isethionic acid, methylmaleic acid, adipic acid, itaconic acid, crotonic acid, oxalic acid, salicylic acid, gallic acid, sorbic acid, gluconic acid and p-oxybenzoic acid as acidic component and with a pulverulent component which is pyrogenic silica, silica sol or precipitated silica, and wherein the water-absorbing polymer structure has at least one of the following properties:

   ($\beta$1) a retention, determined in accordance with ERT 441.2-02, of at least 27 g/g;
   ($\beta$2) an SAP index (SAPI) of at least 140 cm$^3$s/g, the SAP index being defined as follows

$$SAP\ index\ =\ (RET\ x\ SFC)/pH$$

   and wherein

      RET = the retention determined in accordance with ERT 441.2-02,
      SFC = the permeability determined in accordance with the test method described herein and pH = the pH determined in accordance with ERT 400.2-02;

   ($\beta$3) an absorption, determined in accordance with ERT 442.2-02, under a pressure of 50 g/cm$^2$ of at most 20 g/g;
   ($\beta$4) an ammonia-binding capacity, determined in accordance with the test method described herein, of at least 98 mg/g;
   ($\beta$5) a pH, determined in accordance with ERT 400.2-02, of less than 6.5.

7. Foams, shaped articles, fiber, foils, films, cables, sealing materials, liquid-absorbing hygiene articles, carriers for plant and fungal growth-regulating agents, packaging materials, soil additives or building materials comprising the water-absorbing polymer structure according to Claim 6.

8. Use of the water-absorbing polymer structure according to Claim 6 in foams, shaped articles, fibers, foils, films, cables, sealing materials, liquid-absorbing hygiene articles, carriers for plant and fungal growth-regulating agents, packaging materials, soil additives, for controlled release of active compounds, or in building materials.

**Revendications**

1. Procédé pour la préparation d'une structure polymère absorbant l'eau, comprenant les étapes de procédé consistant à :

i) mettre à disposition une structure polymère non traitée, absorbant l'eau, présentant un degré de neutralisation d'au plus 70% en mole ;

ii) mettre en contact la structure polymère non traitée, absorbant l'eau avec un acide organique, choisi dans le groupe constitué par l'acide anisique, l'acide benzoïque, l'acide formique, l'acide valérianique, l'acide citrique, l'acide glyoxylique, l'acide glycolique, l'acide glycérolphosphorique, l'acide glutarique, l'acide chloroacétique, l'acide chloropropionique, l'acide cinnamique, l'acide succinique, l'acide acétique, l'acide tartrique, l'acide lactique, l'acide pyruvique, l'acide fumarique, l'acide propionique, l'acide 3-hydroxypropionique, l'acide malonique, l'acide butyrique, l'acide isobutyrique, l'acide imidinoacétique, l'acide malique, l'acide iséthionique, l'acide méthylmaléique, l'acide adipique, l'acide itaconique, l'acide crotonique, l'acide oxalique, l'acide salicylique, l'acide gallique, l'acide sorbique, l'acide gluconique et l'acide p-oxybenzoïque, comme composant acide,

la structure polymère non traitée, absorbant l'eau, mise à disposition dans l'étape de procédé i) étant postréticulée en surface,
et la structure polymère absorbant l'eau étant mise en contact, dans l'étape de procédé ii), en plus, avec 0,001 à 5% en poids d'un composant inorganique, pour lequel il s'agit de silice pyrogène, de sol de silice ou de silice précipitée, par rapport au poids de la structure polymère non traitée, absorbant l'eau, mise à disposition dans l'étape de procédé i).

**2.** Procédé selon la revendication 1, le degré de neutralisation de la structure polymère non traitée, absorbant l'eau, mise à disposition dans l'étape de procédé i), étant d'au plus 60% en mole.

**3.** Procédé selon la revendication 1 ou 2, le degré de neutralisation de la structure polymère non traitée, absorbant l'eau, mise à disposition dans l'étape de procédé i), étant d'au plus 55% en mole.

**4.** Procédé selon l'une quelconque des revendications précédentes, la structure polymère non traitée, absorbant l'eau étant mise en contact, dans l'étape de procédé ii), avec un fluide $F_2$ contenant le composant acide.

**5.** Procédé selon l'une quelconque des revendications précédentes, la structure polymère non traitée, absorbant l'eau étant mise en contact, dans l'étape de procédé ii), avec 0,1 à 20% en poids du composant acide, par rapport au poids de la structure polymère non traitée, absorbant l'eau, mise à disposition dans l'étape de procédé i).

**6.** Structure polymère absorbant l'eau, pouvant être obtenue par un procédé selon l'une quelconque des revendications 1 à 5, comprenant une zone interne et une zone externe entourant la zone interne, la zone externe de la structure polymère absorbant l'eau ayant été mise en contact avec un acide organique, choisi dans le groupe constitué par l'acide anisique, l'acide benzoïque, l'acide formique, l'acide valérianique, l'acide citrique, l'acide glyoxylique, l'acide glycolique, l'acide glycérolphosphorique, l'acide glutarique, l'acide chloroacétique, l'acide chloropropionique, l'acide cinnamique, l'acide succinique, l'acide acétique, l'acide tartrique, l'acide lactique, l'acide pyruvique, l'acide fumarique, l'acide propionique, l'acide 3-hydroxypropionique, l'acide malonique, l'acide butyrique, l'acide isobutyrique, l'acide imidinoacétique, l'acide malique, l'acide iséthionique, l'acide méthylmaléique, l'acide adipique, l'acide itaconique, l'acide crotonique, l'acide oxalique, l'acide salicylique, l'acide gallique, l'acide sorbique, l'acide gluconique et l'acide p-oxybenzoïque, comme composant acide et avec un composant sous forme de poudre, pour lequel il s'agit de silice pyrogène, de sol de silice ou de silice précipitée, et la structure polymère absorbant l'eau présentant au moins une des propriétés suivantes :

(ß1) une rétention, déterminée selon la norme ERT 441.2-02, d'au moins 27 g/g ;
(ß2) un indice SAP (SAPI) d'au moins 140 cm$^3$ s/g, l'indice SAP étant défini comme suit

$$indice\ SAP = (RET \times SFC)/pH$$

et où

RET = la rétention déterminée selon la norme ERT 441.2-02,
SFC = la perméabilité déterminée selon le procédé de test décrit dans la description et pH = le pH déterminé selon la norme ERT 400.2-02 ;

(ß3) une absorption, déterminée selon la norme ERT 442.2.02 sous une pression de 50 g/cm$^2$, d'au plus 20 g/g ;
(ß4) un pouvoir de fixation de l'ammoniaque, déterminé selon le procédé de test décrit dans la description, d'au moins 98 mg/g ;
(ß5) un pH, déterminé selon la norme ERT 400.2-02, de moins de 6,5.

7. Mousses, corps façonnés, fibres, feuilles, films, câbles, matériaux d'étanchéité, articles hygiéniques absorbant les liquides, supports pour des agents régulant la croissance des plantes et des champignons, matériaux d'emballage, amendements de sol ou matériaux de construction contenant la structure polymère absorbant l'eau selon la revendication 6.

8. Utilisation de la structure polymère absorbant l'eau selon la revendication 6 dans des mousses, des corps façonnés, des fibres, des feuilles, des films, des câbles, des matériaux d'étanchéité, des articles hygiéniques absorbant les liquides, des supports pour les agents régulant la croissance des plantes et des champignons, des matériaux d'emballage, des amendements de sol, pour la libération contrôlée de substances actives ou dans des matériaux de construction.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0113841 A1
- WO 03002623 A1
- US 3794034 A
- WO 0035502 A1
- WO 0132226 A1
- WO 2004037903 A2
- US 4286082 A
- DE 2706135 A1
- US 4076663 A
- DE 3503458 A1
- DE 4020780 C1
- DE 4244548 A1
- DE 4333056 A1
- DE 4418818 A1
- US 4340706 A
- DE 3713601 A1
- DE 2840010 A1
- WO 9605234 A1
- DE 10249821
- WO 02056812 A1
- WO 9526209 A1

**In der Beschreibung aufgeführte Nicht-Patentliteratur**

- **F. L. BUCHHOLZ ; A. T. GRAHAM.** Modern Superabsorbent Polymer Technology. Wiley-VCH, 1998
- **HOLLEMAN ; WIBERG.** Lehrbuch der Anorganischen Chemie. Walter de Gruyter-Verlag, 1985, 750-783